(19) Europäisches Patentamt
European Patent Office
Office européen des brevets

(11) Publication number: 0 226 566
B1

(12) **EUROPEAN PATENT SPECIFICATION**

(45) Date of publication of patent specification: **29.08.90**

(51) Int. Cl.[5]: **C 07 C 51/235**, C 07 C 57/58, C 07 C 57/30, B 01 J 31/04, B 01 J 31/22

(21) Application number: **86870171.5**

(22) Date of filing: **24.11.86**

(54) **Oxidation of 2-arylpropanals.**

(30) Priority: **25.11.85 US 801436**

(43) Date of publication of application:
**24.06.87 Bulletin 87/26**

(45) Publication of the grant of the patent:
**29.08.90 Bulletin 90/35**

(84) Designated Contracting States:
**AT BE CH DE ES FR GB IT LI LU NL SE**

(56) References cited:
**GB-A-1 160 725**

**M. KALEDKOWSKA AND L. NOWAKOWSKI IN ZHURNAL PRIKLADNOI KHIMII, vol. 55, no. 5, May 1982, pages 1116-1121**

**CHEMICAL ABSTRACTS, vol. 56, no. 4, 19th February 1962, column 1, abstract no. 3339a, Columbus, Ohio, US; H. HOCK et al. "Heavy metal catalysis of the autoxidation of aldehydes"; & Journal der praktischen Chemie, 1961, (14), 72-83**

(73) Proprietor: **MONSANTO COMPANY**
**Patent Department 800 North Lindbergh Boulevard**
**St. Louis, Missouri 63167 (US)**

(72) Inventor: **Getman, Daniel Paul**
**12944 Summit Ridge Drive**
**St. Louis Missouri 63146 (US)**
Inventor: **Beck, Gary Robert**
**1417 Mirandy Drive**
**St. Louis Missouri 63146 (US)**
Inventor: **Riley, Dennis Patrick**
**15519 Country Ridge Drive**
**Chesterfield Missouri 63017 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

**EP 0 226 566 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
PATENT ABSTRACTS OF JAPAN, vol. 3, no. 7 (C-34), 24th January 1979, page 92; & JP - A - 53 130 629 (MITSUBISHI GAS KAGAKU) 14-11-1978

PATENT ABSTRACTS OF JAPAN, vol. 8, no. 255 (C-253)1692r, 21th November 1984; & JP - A - 59 134 737 (MITSUBISHI KASEI KOGYO) 08-02-1984

PATENT ABSTRACTS OF JAPAN, vol. 7, no. 97 (C-163)1242r, 23th April 1983; & JP - A - 58 21642 (TORAY K.K.) 02-08-1983

**Description**

The present invention concerns oxidation of 2-arylpropionaldehydes to 2-arylpropionic acids which are known to have useful therapeutic properties. In particular, the invention is directed to a method of oxidizing 2-(4-isobutylphenyl)propionaldehyde to 2-(4-isobutylphenyl)propionic acid by molecular oxygen in a solvent medium containing a manganese catalyst.

Background of the invention

Aryl propionic acids have been recognized as therapeutic compounds, with one of the more prominent being 2-(4-isobutylphenyl)propionic acid, known as ibuprofen and widely used as an analgesic and as an anti-inflammatory agent. One of the known procedures for preparing ibuprofen involves oxidation of 2-(4-isobutylphenyl)propionaldehyde by reaction with silver oxide, see British Patent 1,160,725. Silver oxide is a relatively expensive reactant which it is therefore necessary to recover for recycle. Another procedure involves oxidation with molecular oxygen employing a cobalt or silver catalyst, but selectivity to the desired product is not good; see Chemical Abstracts, Vol. 78, page 110916w, citing Japanese Patent 72 39 051 (December 1972). Another procedure uses light as catalyst, see Chemical Abstracts Vol. 86, page 89399h, citing Japanese Patent 76 100,042.

The use of manganese catalysts in the oxidation of isobutyraldehyde to isobutyric acid in the liquid phase is disclosed in Zhurnal Prikladnoi Khimii, Vol. 55 (1982) pp. 1116—1121 (English translation pp. 1034—1038). The optimum temperature is said to be 50—55°C.

The present invention provides a method of preparing a 2-arylpropionic acid which comprises oxidizing a 2-arylpropional with molecular oxygen in a solvent in the presence of a catalyst, characterised in that the solvent is a non-polar solvent, the catalyst comprises a manganese catalyst, and the oxidation is carried out at a temperature of 25°C or below and at an oxygen pressure of at least 172.38 kPa.

The present invention involves an oxidation employing molecular oxygen and catalyst to convert 2-arylpropionaldehydes to 2-arylpropionic acid, as represented:

$$R\overset{\displaystyle CH_3}{\overset{|}{C}}HCHO \rightarrow R\overset{\displaystyle CH_3}{\overset{|}{C}}HCOOH$$

in which R represents an aryl group, which can be substituted or unsubstituted, varying from an unsubstituted phenyl group to substituted or polynuclear aryl compounds, with those being of particular interest in which the arylpropionic acid product has therapeutic properties; for example, R can represent p-isobutylphenyl, 6-chlorocarbazyl-2, 3-phenoxyphenyl, 2-isopropylindanyl-5, 2-fluoryl, 2-fluoro-4-biphenyl, and 6-methoxynaphthyl-2. The aforesaid British Patent 1,160,725, the disclosure of which is incorporated herein by reference, describes various other arylpropionaldehydes which can be oxidized to arylpropionic acids having therapeutic properties. In some of the compounds of interest, R represents phenyl or substituted phenyl, such as 4-alkylphenyl, 4-(2-fluorophenyl)phenyl, 3-phenoxyphenyl or phenyl itself. Oxidations yielding 2-(4-isobutylphenyl)propionic acid are of particular interest, and illustrations of that reaction will be utilized herein as exemplifications of the invention.

Manganese salts have been found particularly advantageous for use as catalysts in the oxidation of 2-(4-isobutylphenyl)propionaldehyde by molecular oxygen to the corresponding acid, as such catalysts have good activity and give high selectivity to the desired product, such as 85% conversion in two hours with 85% selectivity to desired product. It is also significant that use of a manganese catalyst makes it feasible to limit production of an undesired formate product to very low amounts, in contrast to usual results with cobalt catalysts. A soluble cobalt catalyst can however be utilized in conjunction with the manganese catalyst. In oxidations utilizing cobalt alone as catalyst, 1-(p-isobutylphenyl)ethyl formate is generated in substantial amounts, constituting as much as 15 to 25% of product. However, by employing a manganese catalyst, particularly in soluble form as taught herein, it is possible to virtually eliminate the formate production. The formate is believed to arise from a Baeyer-Villiger reaction (J. March, "Advanced Organic Chemistry, Reactions, Mechanisms and Structure", 2nd Ed., McGraw-Hill, 1977, pg. 1011) of a peroxy acid, 2-(4-isobutylphenyl)propionic peracid, RC(O)OOH, with the starting aldehyde. It appears that the manganese catalyst produces manganese in suitable form, possibly Mn(III) in equilibrium with other forms, to catalytically reduce the peroxy acid to the desired substituted propionic acid, thereby avoiding production of the formate. Regardless of what the mechanism may be, it has been demonstrated that the manganese catalyst is advantageous in minimizing yield losses to formate. Moreover, when the manganese is used in soluble form in homogeneous media, it is believed to be more readily available to react and avoid the undesirable pathways.

Another side-product produced in the oxidation of 2-(p-isobutyl)propionaldehyde is a ketone, p-isobutylacetophenone. This ketone can be recycled in a preparative scheme, as the 2-(p-isobutyl)propionaldehyde can be produced in a step-wise procedure from the corresponding acetophenone, as cited in British Patent 1,160,725. However, it is desirable to minimize production of the ketone in the preparation of the acid. While the ketone may constitute 10 mole % or so of the final reaction mixture, it has been found that the ketone is produced primarily during the initiation stage of the oxidation,

and thereafter the ketone concentration remains relatively constant. If ketone is added initially it does not significantly affect results. During a relatively long initiation period with manganese catalysts, such as 1 hour, the aldehyde reactant is slowly consumed generating ketone. The subsequent catalytic reaction is then very selective to 2-(p-isobutylphenyl)propionic acid, the selectivity approaching 97% when the deleterious initiation reaction is factored out. Thus it is considered feasible to use a pre-generated catalyst and to eliminate the deleterious initiation period in which aldehyde is consumed while the actual catalyst is being generated. It is also possible to add peracids as initiators, thereby shortening the catalyst generation time, and lessening the amount of ketone produced. Another variation is to initially add an inexpensive sacrificial adehyde, such as benzaldehyde, for the catalyst generation stage. Additionally, the catalyst can in effect be recycled by adding additional 2-(p-isobutylphenyl)propionaldehyde, preferably in amounts to maintain the optimum reaction rate. It appears that the manganese catalyzed oxidations slow down because of kinetic consequences, with a particular decline in rate being evident at about 0.04 molar concentration or lower of the aldehyde reactant. Thus while conversions in an initial cycle do not generally exceed 85%, indications are that the use of several cycles can raise the conversion to over 99%. The selectivities obtainable are over 85%, with the major side-product being 4-isobutylacetophenone and as discussed hereinabove, provisions can be taken to lessen the amount of that product. The catalyst, once generated, can be used continually and its activity is not affected by the build-up of the product or side-products.

The present process is carried out in a non-polar solvent medium, temperatures of 25°C or below. Usual non-polar solvents for organic reactions can be employed, with a preference for hydrocarbon and similar solvents. Thus such solvents can be employed as aliphatic and aromatic hydrocarbons, e.g. alkanes of 5 to 12 or so carbon atoms or toluene, or chlorobenzene.

In the present process, the catalysts are preferably utilized in soluble form so that the oxidation is carried out in a homogeneous system. This is particularly important in the case of manganese catalyst as the use of a soluble catalyst contributes to higher selectivity of the desired propionic acid product. Manganese and cobalt readily form salts with organic acids and various soluble salts can be prepared. Salts of relatively long chain fatty acids, such as those of about 8 to 20 or so carbon atoms, will generally have the requisite solubility in hydrocarbon and similar solvents and can be used. For example, manganese and cobalt stearates, manganese and cobalt decanoates, manganese and cobalt palmitates, etc. can be used. Other soluble salts or complexes with organic compounds can similarly be used, e.g. manganese and cobalt acetyl acetonates and manganese and cobalt ibuprofenates. The acetylacetonates are at times herein, particularly in tables, designated by "acac". Having the catalyst, as well as the aldehyde reactant, in solution contributes to obtaining consistent and reproducible results and faster reaction rates. In addition, the process operated as a homogeneous system is adaptable to continuous operation, as additional aldehyde can be added continuously or at intervals, and the product tends to precipitate, and the precipitate can be removed as convenient.

The process involves oxidation with oxygen, and operation with elevated oxygen pressure of at least 172.38 kPa contributes to selectivity to the desired product. Preferably the oxygen pressure is at least 50 psi (344.75 kPa) with some possible additional improvement in results at pressures up to 150 psi (1033.75 kPa) or so. Higher pressures can be used up to 1000 psi or higher (6,895 kPa) but generally without additional benefit and possibly with higher costs. Oxygen can, if desired, be supplied in admixture with other gases, as in air or other sources.

The temperature of the process has a marked effect upon results. Elevated temperatures contribute to side reactions, so zhat the oxidation in accordance with the invention is carried out at a temperature of 25°C or below. For example, good results can be obtained at temperatures below about 20°C, preferably no greater than about 10°C, such as in the range of about −10°C to about +10°C. Still lower temperatures can be employed, but usually without significant additional advantage.

Additional additives can be used with the catalyst; sodium salts have been found to be useful in conjunction with catalysts containing cobalt e.g. sodium salts of various carboxylic acids, such as sodium stearate and sodium ibuprofenate. Under some conditions sodium ibuprofenate itself appears to function as a catalyst, but this does not appear to be the case when possible metal contaminants are carefully excluded from a reaction system.

The manganese and cobalt catalyst components can be added as such, or generated in situ. The active manganese forms appear to be Mn(II) and Mn(III) with Mn(IV) probably being in equilibrium. As the manganese is oxidized and reduced as it takes part in various aspects of the reaction, it can be added as Mn(II) or Mn(III) salts, for example, as Mn(II)stearate, Mn(III)stearate, Mn(II)acetylacetonate or Mn(III)acetylacetonate. Similarly cobalt can be added as cobalt(II) or (III). While there is advantage in using manganese in soluble form as discussed hereinabove, it can also be employed in insoluble forms, such as manganese acetate.

Example 1

A number of oxidation runs were made in a Fischer Proter bottle equipped with a mixing pump. A 0.22 millimole amount of catalyst was used with 1.01 grams (5.3 mmol) of 2-(4-isobutylphenyl)propanol and 4.02 grams decane as solvent. The bottle was immersed in an ice bath, purged with oxygen and then charged with oxygen to designated pressure. As the oxygen was consumed, more was added to maintain

the pressure. After a two-hour reaction time, a heavy, white precipitate of ibuprofen had formed. The bottle was vented, opened and approximately 4 grams methylene chloride was added to dissolve the white solids. A sample was withdrawn, silylated, and analyzed by chromatography, based on uncalibrated peak areas and percent of the peaks observed. Results are reported in Table 1 for runs conducted with manganese and cobalt catalysts at different temperatures. It is evident that the selectivity to ibuprofen improved at lower temperatures. The cobalt and manganese catalysts were used as acetate salts.

TABLE 1

| Runs | Catalyst | psi $O_2$ | Rxm temp. | % Conversion | % Selectivity |
|---|---|---|---|---|---|
| 1** | Co | 120* | 25 | 83 | 46 |
| 2** | Co | 120 | 0 | 80 | 53 |
| 3 | Co/Mn | 60* | 50 | 98 | 53 |
| 4 | Co/Mn | 60 | 25 | 92 | 62 |
| 5 | Co/Mn | 60 | 0 | 92 | 76 |
| 6 | Mn | 120 | 25 | 93 | 66 |
| 7 | Mn | 120 | 0 | 92 | 74 |

* (120 psi is 827.4 kPa and 60 psi is 413.7 kPa).
**Comparative runs.

Example 2

A number of oxidations were conducted as in Example 1 but at varying pressures, with results in Table 2.

TABLE 2

| Runs | Catalyst | psi $O_2$ | Rxm temp. | % Conversion | % Selectivity |
|---|---|---|---|---|---|
| 8** | Co | 10* | 25 | 62 | 34 |
| 9** | Co | 60* | 25 | 86 | 53 |
| 10** | Co | 120* | 25 | 83 | 46 |
| 11 | Co/Mn | 60 | 25 | 92 | 62 |
| 12 | Co/Mn | 120 | 25 | 94 | 63 |
| 13 | Co/Mn | 60 | 0 | 92 | 76 |
| 14 | Co/Mn | 120 | 0 | 87 | 79 |
| 15 | Mn | 60 | 25 | 96 | 57 |
| 16 | Mn | 120 | 25 | 93 | 66 |

*(10 psi is 68.95 kPa, 60 psi is 413.7 kPa and 120 psi is 827.4 kPa).
**Comparative runs.

Example 3

A number of oxidations were conducted as in Example 1, employing different solvents, with results as reported in Table 3.

TABLE 3

| Runs | Catalyst | psi $O_2$ | Rxm temp. | Solvent | % Conversion | % Selectivity |
|---|---|---|---|---|---|---|
| 17 | Co/Mn | 120* | 0°C | Toluene | 85 | 75 |
| 18 | Co/Mn | 120 | 0°C | Prop. acid | 52 | 60 |
| 19 | Co/Mn | 120 | 0°C | Decane | 87 | 79 |

*(120 psi is 827.4 kPa).

Example 4

A number of oxidation runs were made in a Fischer Porter bottle equipped with a micro gas mixing pump. In typical procedures, the bottle was charged with 0.12 mole catalyst, 1.7 mol 2-(4-isobutylphenyl)propionaldehyde of 94.2% purity, 2.3 cc dodecane (internal standard) and 16 cc decane. The bottle was immersed in the salt bath to cool to −10°C, purged three times with oxygen at 60 psi gauge (413.7 kPa gauge) and then raised to a pressure of 120 psi gauge oxygen (827.4 kPa gauge). As oxygen was consumed, additional oxygen was added to maintain pressure. At the end of the reaction period, the gas was vented and 5 cc methylene chloride was added to dissolve the ibuprofen product. For analysis, a sample was silated with Regisil®-RC-3 silating material (bis(trimethylsilyl)trifluoroacetamide plus 10% trimethylchlorosilane) with 0.1 cc sample to 0.5 cc of silating material. A gas chromatograph equipped with a methylsilicone capillary column was used for the analysis. The results using various catalysts at a 6 millimolar concentration and generally three-hour time are set forth in Table 4, showing the selectivity to ibuprofen (Ibu) and the percentages of formate, ketone and alcohol by-products. The 2-(4-isobutylphenyl)propionaldehyde reactant was employed at a 0.4 M concentration. The high selectivities to the desired ibuprofen with the various soluble manganese compounds are notable, along with the substantial avoidance of formate production. In the table, acac refers to the acetylacetonates.

TABLE 4

| Catalyst | % Conv. | Select to Ibu | Molar recovery | % Formate | % Ketone | % Alcohol |
|---|---|---|---|---|---|---|
| **Co(II)acac[a] | 87 | 57 | 98 | 24 | | 2 |
| **Co(III)acac | 79 | 53 | 94 | 23 | 7 | 1.5 |
| **Co(II)stearate[a] | 96 | 63 | 97 | 15 | 14 | 5 |
| **Fe(II)stearate | 35 | 39 | 96 | 8 | 7 | 3 |
| **Fe(III)acac | 73 | 42 | 80 | 10.5 | 5 | 5 |
| **Ni(II)stearate | 24 | 49 | 98 | 8 | 2 | — |
| **Ni(II)acac | 61 | 57 | 94 | 15 | 4 | 1.5 |
| **Cr(III)2-EtHeX | 41 | 25 | 79 | 3 | 6 | 2 |
| **Cr(III)acac | 4 | 22 | 99 | | | |
| **Cu(II)acac | 61 | 57 | 94 | 15 | 4 | 1.5 |
| Mn(II)stearate | 82 | 75 | 95 | 4 | 9 | 1 |
| Mn(II)acac | 81 | 79 | 100 | 1 | 12 | 2 |
| Mn(III)acac | 78 | 84.5 | 100 | 0 | 12 | 1 |
| Mn(II)TPP[b] | 78 | 79 | 95 | 0.1 | 11 | 1 |

[a]Five hours.
[b]Tetraphenylporphyrin.
**Comparative runs.

Example 5

Oxidations were conducted with 0.40 M concentrations of 2-(4-isobutylphenyl)propionaldehyde in various solvents, with results, as reported in Table 5.

TABLE 5

| Catalyst[a] | Solvent | % Cov. | % Select. | Molar rec. |
|---|---|---|---|---|
| Co(II)acac[b] | acetone | 33 | 33 | 96 |
| Mn stearate[c] | acetone | 21 | 51 | 95 |
| Mn stearate[d] | toluene | 63 | 76 | 96 |
| Mn stearate[d] | chlorobenzene | 67 | 72 | 96 |
| Mn stearate | 1:1 decane/aldehyde | 53°C exotherm (−10 to 43°C) 79 | 67 | 90 |

[a]Cat=6 mM.
[b]Five hrs, Comparative result.
[c]2.5 hrs.
[d]Two hrs.
$PO_2$=120 psi (827.4 kPa) at 0°C.

Example 6

Several oxidations were conducted with 0.40 M concentrations of 2-(4-isobutylphenyl)propionaldehyde in decane under 120 psi oxygen, with reaction initiators along with the catalyst, the initiators being benzaldehyde and m-chloroperoxybenzoic acid (MCPBA). Results are reported in Table 6.

TABLE 6
Initiator effects

| Catalyst | Initiator | Time (hrs) | % Conv. | Select. | Molar rec. | % Ketone |
|---|---|---|---|---|---|---|
| Mn stearate[a] | MCPBA[b] | 1[c] | 75 | 81 | 96 | 7.5[d] |
| | | 2 | 80 | 85 | 97 | 7.6 |
| Mn stearate[a] | benzaldehyde[e] | 2[f] | 81 | 82 | 96 | 9[d] |
| Mn stearate[g] | MCPBA[h] | 2[i] | 68 | 84 | 95 | 3.2[d] |

[a][Mn]=6 mM.
[b][In]=6.0 mM.
[c]Ten min to initiate at −10°C.
[d]No formate or alcohol generated.
[e][benz.]=16.0 mM.
[f]30 min to exotherm at −10°C.
[g][Mn]=3.0 mM, [aldehyde]=0.2 M.
[h][In]=3.0 mM.
[i]Twenty min to exotherm at −10°C [1]=0.40 *M* in decane under 120 psi (827.4 kPa) $O_2$.

**Claims**

1. A method of preparing a 2-arylpropionic acid which comprises oxidizing a 2-arylpropional with molecular oxygen in a solvent in the presence of a catalyst, characterised in that the solvent is a non-polar solvent, the catalyst comprises a manganese catalyst, and the oxidation is carried out at a temperature of 25°C or below and at an oxygen pressure of at least 172.38 kPa.

2. The method of Claim 1 wherein the manganese catalyst is in the form of a soluble organic salt or a complex.

3. The method of either of Claims 1 and 2 wherein a soluble organic cobalt salt or complex is utilized in conjunction with the manganese catalyst.

4. The method of any of Claims 1 to 3 wherein the 2-arylpropional is a 2-(4-alkylphenyl)propionaldehyde.

5. The method of Claim 4 wherein the arylpropional is 2-(4-isobutylphenyl)propionaldehyde.

6. The method of Claim 5 wherein an additional amount of 2-(4-isobutylphenyl)propionaldehyde is added after the reaction has commenced.

7. The method of either of Claims 5 and 6 wherein an initiator is utilized to lessen the catalyst induction period and to lessen the amount of p-isobutylacetophenone produced during induction.

8. The method of any of Claims 1 to 3 wherein the arylpropional is 2-phenylpropionaldehyde.

9. The method of Claim 3 wherein a sodium carboxylic acid salt is also present.

**Patentansprüche**

1. Verfahren zur Herstellung einer 2-Arylpropionsäure, welches das Oxidieren eins 2-Arylpropanals mit molekularem Sauerstoff in einem Lösungsmittel in Anwesenheit eines Katalysators umfaßt, dadurch gekennzeichnet, daß das Lösungsmittel ein nichtpolares Lösungsmittel ist, der Katalysator einen Mangankatalysator umfaßt und die Oxidation bei einer Temperatur von 25°C oder niedriger und bei einem Sauerstoffdruck von mindestens 172,38 kPa durchgeführt wird.

2. Verfahren nach Anspruch 1, worin der Mangankatalysator in Form eines löslichen organischen Salzes oder eines Komplexes ist.

3. Verfahren nach einem der Ansprüche 1 und 2, worin ein lösliches organisches Kobaltsalz oder -komplex in Verbindung mit dem Mangankatalysator verwendet wird.

4. Verfahren nach einem der Ansprüche 1 bis 3, worin das 2-Arylpropanal ein 2-(4-Alkylphenyl)-propionaldehyd ist.

5. Verfahren nach Anspruch 4, worin das Arylpropanal 2-(4-Isobutylphenyl)-propionaldehyd ist.

6. Verfahren nach Anspruch 5, worin eine zusätzliche Menge 2-(4-Isobutylphenyl)-propionaldehyd nach dem Einsetzen der Reaktion zugegeben wird.

7. Verfahren nach einem der Ansprüche 5 und 6, worin ein Initiator verwendet wird, um die Katalysatorinduktionsperiode zu verkürzen und die Menge des während der Induktion gebildeten p-Isobutylacetophenons zu verringern.

8. Verfahren nach einem der Ansprüche 1 bis 3, worin das Arylpropanal 2-Phenylpropionaldehyd ist.

9. Verfahren nach Anspruch 3, worin auch ein Natriumcarbonsäuresalz vorhanden ist.

**Revendications**

1. Procédé pour préparer un acide 2-arylpropionique qui comprend l'oxydation d'un 2-arylpropional avec de l'oxygène moléculaire dans un solvant en présence d'un catalyseur, caractérisé en ce que le solvant est un solvant non polaire, le catalyseur comprend un catalyseur en manganèse, et l'oxydation est effectuée à une température de 25°C ou à une température inférieure et à une pression de l'oxygène d'au moins 172,38 kPa.

2. Procédé selon la revendication 1, dans lequel le catalyseur en manganèse se présente sous la forme d'un sel organique soluble ou d'un complexe.

3. Procédé selon la revendication 1 ou la revendication 2, dans lequel on utilise un sel de cobalt organique soluble ou un complexe en conjonction avec le catalyseur en manganèse.

4. Procédé selon l'une quelconque des revendications 1 à 3, dans lequel le 2-arylpropional est un 2-(4-alkylphényl)propionaldéhyde.

5. Procédé selon la revendication 4, dans lequel l'arylpropional est le 2-(4-isobutylphényl)propionaldéhyde.

6. Procédé selon la revendication 5, dans lequel on ajoute une quantité supplémentaire de 2-(4-isobutylphényl)propionaldéhyde après le commencement de la réaction.

7. Procédé selon l'une ou l'autre des revendications 5 et 6, dans lequel on utilise un initiateur pour abaisser la période d'induction du catalyseur et pour réduire la quantité de p-isobutylacétophénone produite pendant l'induction.

8. Procédé selon l'une quelconque des revendications 1 à 3, dans lequel l'arylpropional est le 2-phénylpropionaldéhyde.

9. Procédé selon la revendication 3, dans lequel un sel de sodium de l'acide carboxylique est également présent.